# EUROPEAN PATENT SPECIFICATION

(11) **EP 3 493 875 B1**
(45) Date of publication and mention of the grant of the patent: **27.05.2020**
(21) Application number: 17791830.7
(22) Date of filing: 13.10.2017
(51) Int. Cl.: A61N 1/05, A61N 1/372, G06T 7/73, A61B 6/03, A61B 6/12, A61B 90/00

(54) **SYSTEMS AND METHODS FOR DETERMINING ORIENTATION OF AN IMPLANTED LEAD**
SYSTEME UND VERFAHREN ZUR BESTIMMUNG DER AUSRICHTUNG EINER IMPLANTIERTEN ELEKTRODE
SYSTÈMES ET PROCÉDÉS PERMETTANT DE DÉTERMINER L'ORIENTATION D'UN FIL IMPLANTÉ

(30) Priority: 14.10.2016 US 201662408399 P
(43) Date of publication of application: 12.06.2019
(73) Proprietor: Boston Scientific Neuromodulation Corporation, Valencia, CA 91355 (US)
(72) Inventor: BOKIL, Hemant, Santa Monica, CA 90402 (US)
(74) Representative: Vossius & Partner Patentanwälte Rechtsanwälte mbB
(86) International application number: PCT/US2017/056627
(87) International publication number: WO 2018/071842

(56) References cited:
- WO-A1-2014/151651
- US-A1- 2009 287 271
- US-A1- 2010 135 553
- US-A1- 2012 016 378
- US-A1- 2013 039 550

## Description

### FIELD

The present invention is directed to the area of implantable electrical stimulation systems and methods of making and using the systems. The present invention is also directed to systems and methods for determining the orientation of an implanted electrical stimulation lead.

### BACKGROUND

Implantable electrical stimulation systems have proven therapeutic in a variety of diseases and disorders. For example, spinal cord stimulation systems have been used as a therapeutic modality for the treatment of chronic pain syndromes. Peripheral nerve stimulation has been used to treat chronic pain syndrome and incontinence, with a number of other applications under investigation. Functional electrical stimulation systems have been applied to restore some functionality to paralyzed extremities in spinal cord injury patients. Stimulation of the brain, such as deep brain stimulation, can be used to treat a variety of diseases or disorders.

Stimulators have been developed to provide therapy for a variety of treatments. A stimulator can include a control module (with a pulse generator), one or more leads, and an array of stimulator electrodes on each lead. The stimulator electrodes are in contact with or near the nerves, muscles, or other tissue to be stimulated. The pulse generator in the control module generates electrical pulses that are delivered by the electrodes to body tissue.

US 2013/039550 A1 relates to a system for generating an anatomical atlas, the system comprising: a computer processor configured to: for each patient of a patient population: obtain a respective anatomical atlas; and register the respective anatomical atlas to an anatomical image of a current patient to obtain a respective registered anatomical atlas; and determine an average of the registered anatomical atlases.

US 2009/287271 A1 relates to a method of a volume of activation (VOA) providing machine to provide a VOA of a stimulation electrode leadwire, comprising: calculating, by a processor of the machine, a VOA for each of a plurality of sets of parameter settings of the leadwire; storing in a database each of the calculated VOAs in association with the respective set of parameter settings for which it was calculated; obtaining a further set of parameter settings of the leadwire; determining, by the processor, a VOA for the obtained set of parameter settings based on the stored VOAs; and outputting, by the machine, the determined VOA.

US 2010/135553 A1 relates to a method comprising: obtaining a two dimensional (2D) image of electrodes on a lead implanted within a patient; determining actual 2D coordinates of the electrodes in the 2D image; obtaining 2D coordinates projected from 3D coordinates of electrodes on a selected type of lead; comparing the projected 2D coordinates to the actual 2D coordinates; and determining a characterization of the implanted lead based on the comparison.

### BRIEF SUMMARY

The present invention is defined by the claims.

One embodiment is a method for identifying a rotational orientation of an implanted electrical stimulation lead. The method includes obtaining a plurality of radiological images of the lead generated using an imaging technique, the lead including a lead body, a plurality of segmented electrodes disposed along a distal portion of the lead body, and an asymmetric marker disposed along the distal portion of the lead body and including a longitudinal band that extends around a portion of a circumference of the lead body and defines a marker window extending around a remainder of the circumference of the lead body and opposite the longitudinal band, wherein the asymmetric marker and lead body are distinguishable from each other in the radiological images (for example, the asymmetric marker and lead body are formed of different materials), wherein each segmented electrode extends around no more than 50% of a circumference of the lead body. The method also includes generating an isosurface image from the plurality of radiological images and displaying the isosurface image on a display device, wherein the isosurface image includes an image of the longitudinal band of the marker; identifying a bulge in the isosurface image corresponding the longitudinal band of the marker; determining a predicted rotational orientation of the lead based on the bulge in the isosurface image (for example, on the position or shape or both position and shape of the bulge); and determining a rotational orientation of the lead by correcting the predicted rotational orientation of the lead based on at least one parameter of the imaging technique used to generate the radiological images. In at least some embodiments, the method further includes displaying, on the display device, a model of at least the distal portion of the lead oriented along the rotational orientation. In at least some embodiments, the method further includes rotating the isosurface image on the display device in response to a user command.

Another embodiment is a method for identifying a rotational orientation of an implanted electrical stimulation lead. The method includes receiving image data of at least a portion of the lead including image data of the marker, wherein the image data is generated using an imaging technique; receiving at least one template of the lead having a specified rotational orientation, the at least one template including (1) a reference data cube (X_{R}) of the marker and (2) a reference marker direction vector (*v*_{R}) indicative of the specified rotational orientation of the lead about the longitudinal axis; producing a target data cube (X_{T}) of the marker using the image data of the marker; registering the reference data cube (X_{R}) to the target data cube (X_{T}) to produce a transformation operator (Φ) for transforming X_{R} into a transformed reference data cube Φ(X_{R}) that matches X_{T} within a specified margin; estimating a predicted rotational orientation of the lead using the reference marker direction vector (*v*_{R}) and the determined transformation operator (Φ); and determining a rotational orientation of the lead by correcting the predicted rotational orientation of the lead based on at least one parameter of the imaging technique used to generate the image data. In at least some embodiments, estimating the predicted rotational orientation of the lead includes applying the determined transformation operator (Φ) to the reference direction vector (*v*_{R}) to produce an estimated marker direction vector (*ṽ_{T}*) indicative of the predicted rotational orientation of the lead relative to an imaging axis used for producing the image data of the at least a portion of the lead. In at least some embodiments, registering the reference data cube (X_{R}) to the target data cube (X_{T}) includes performing a multi-atlas registration of respective reference data cubes associated with the two or more templates to the target data cube, to produce respective transformation operators corresponding to the two or more templates; and estimating the predicted rotational orientation of the lead includes estimating the predicted rotational orientation using a combination of the two or more estimated marker direction vectors estimated using reference direction vectors and respective transformation operators.

In at least some embodiments, the at least one template of the lead having a specified rotational orientation is generated from an actual image of a lead or from a simulated image of a lead. In at least some embodiments of the methods described above, the at least one parameter includes an angle of the lead with respect to scan planes of the imaging technique. In at least some embodiments of the methods described above, correcting the predicted rotational orientation includes applying a bias term to the predicted rotational orientation based on the angle of the lead with respect to the scan planes of the imaging technique.

In at least some embodiments of the methods described above, the at least one parameter includes an angle of the asymmetric marker with respect to scanner coordinate axes of the imaging technique. In at least some embodiments of the methods described above, correcting the predicted rotational orientation includes applying a bias term to the predicted rotational orientation based on the angle of the asymmetric marker with respect to the scanner coordinate axes of the imaging technique. In at least some embodiments of the methods described above, applying the bias term includes obtaining the bias term from a database. In at least some embodiments of the methods described above, obtaining the bias term from a database includes determining the bias term by interpolating entries of the database.

In at least some embodiments of the methods described above, correcting the predicted rotational orientation includes correcting the predicted rotational orientation using a relationship between the predicted rotational orientation and the angle of the lead with respect to the scan planes of the imaging technique determined by imaging leads at known orientations using the imaging technique.

In at least some embodiments of the methods described above, the at least one parameter includes a slice thickness of the imaging technique. In at least some embodiments of the methods described above, the at least one parameter includes a pitch of the scan planes of the imaging technique.

Yet another embodiment is a system for identifying a rotational orientation of an implanted electrical stimulation lead, the system including a computer processor configured and arranged to perform any of the methods described above.

A further embodiment is a non-transitory computer-readable medium having computer executable instructions stored thereon that, when executed by a processor, cause the processor to perform any of the methods described above.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic view of one embodiment of an electrical stimulation system, according to the invention;
FIG. 2 is a schematic side view of one embodiment of an electrical stimulation lead, according to the invention;
FIG. 3 is a schematic diagram of one embodiment of the distal portion of a lead with an asymmetric marker, according to the invention;
FIG. 4A is a schematic diagram of one embodiment of an isosurface image, according to the invention;
FIG. 4B is a schematic diagram of another embodiment of an isosurface image, according to the invention;
FIG. 5A is a schematic diagram of a portion of the isosurface image of Figure 4A, according to the invention;
FIG. 5B is a schematic diagram of a portion of the isosurface image of Figure 4B, according to the invention;
FIG. 6 is a flowchart of one embodiment of a method of determining corrections to predicted rotational orientations of a lead, according to the invention;
FIG. 7 is a schematic block diagram of one embodiment of a system for determining rotational orientation of a lead, according to the invention;
FIG. 8A is a schematic diagram of the portion of the isosurface image of Figure 5A at a different angle, according to the invention;
FIG. 8B is a schematic diagram of a portion of the isosurface image of Figure 5A from a top-down view, according to the invention;
FIG. 9 is a flowchart of one embodiment of another method of determining rotational orientation of a lead, according to the invention;
FIG. 10 is a flowchart of one embodiment of another method of determining rotational orientation of a lead, according to the invention; and
FIG. 11 is a flowchart of one embodiment of yet another method of determining rotational orientation of a lead, according to the invention.

### DETAILED DESCRIPTION

The present invention is directed to systems and methods for determining the orientation of an implanted electrical stimulation lead.

Suitable implantable electrical stimulation systems include, but are not limited to, a least one lead with one or more electrodes disposed on a distal end of the lead and one or more terminals disposed on one or more proximal ends of the lead. Leads include, for example, percutaneous leads, paddle leads, cuff leads, or any other arrangement of electrodes on a lead. Examples of electrical stimulation systems with leads are found in, for example, U.S. Patents Nos. 6,181,969; 6,516,227; 6,609,029; 6,609,032; 6,741,892; 7,244,150; 7,450,997; 7,672,734;7,761,165; 7,783,359; 7,792,590; 7,809,446; 7,949,395; 7,974,706; 8,175,710; 8,224,450; 8,271,094; 8,295,944; 8,364,278; 8,391,985; and 8,688,235; and U.S. Patent Applications Publication Nos. 2007/0150036, 2009/0187222; 2009/0276021; 2010/0076535; 2010/0268298; 2011/0005069; 2011/0004267; 2011/0078900; 2011/0130817; 2011/0130818; 2011/0238129; 2011/0313500; 2012/0016378; 2012/0046710; 2012/0071949; 2012/0165911; 2012/0197375; 2012/0203316; 2012/0203320; 2012/0203321; 2012/0316615; 2013/0105071; and 2013/0197602. In the discussion below, a percutaneous lead will be exemplified, but it will be understood that the methods and systems described herein are also applicable to paddle leads and other leads.

A percutaneous lead for electrical stimulation (for example, deep brain or spinal cord stimulation) includes stimulation electrodes that can be ring electrodes, segmented electrodes that extend only partially around the circumference of the lead, or any other type of electrode, or any combination thereof. The segmented electrodes can be provided in sets of electrodes, with each set having electrodes circumferentially distributed about the lead at a particular longitudinal position. For illustrative purposes, the leads are described herein relative to use for deep brain stimulation, but it will be understood that any of the leads can be used for applications other than deep brain stimulation, including spinal cord stimulation, peripheral nerve stimulation, dorsal root ganglion stimulation, or stimulation of other nerves, muscles, and tissues.

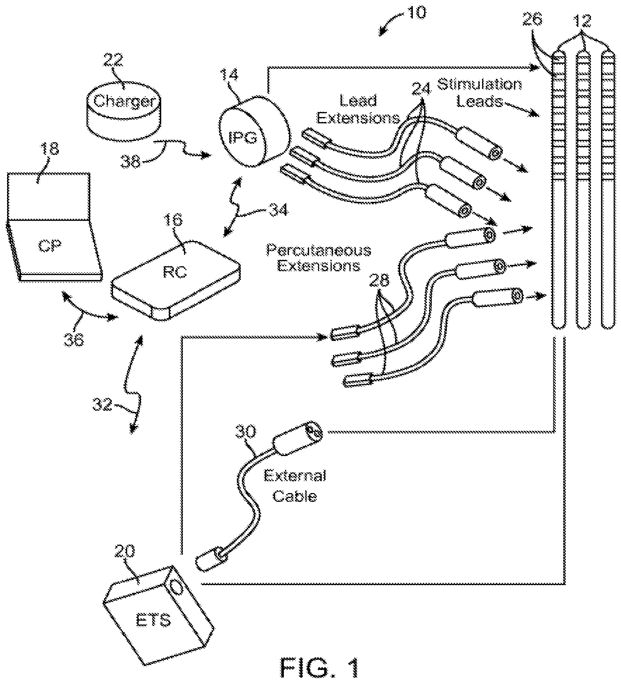

Turning to Figure 1, one embodiment of an electrical stimulation system 10 includes one or more stimulation leads 12 and an implantable pulse generator (IPG) 14. The system 10 can also include one or more of an external remote control (RC) 16, a clinician's programmer (CP) 18, an external trial stimulator (ETS) 20, or an external charger 22.

The IPG 14 is physically connected, optionally via one or more lead extensions 24, to the stimulation lead(s) 12. Each lead carries multiple electrodes 26 arranged in an array. The IPG 14 includes pulse generation circuitry that delivers electrical stimulation energy in the form of, for example, a pulsed electrical waveform (i.e., a temporal series of electrical pulses) to the electrode array 26 in accordance with a set of stimulation parameters. The implantable pulse generator can be implanted into a patient's body, for example, below the patient's clavicle area or within the patient's buttocks or abdominal cavity. The implantable pulse generator can have eight stimulation channels which may be independently programmable to control the magnitude of the current stimulus from each channel. In some embodiments, the implantable pulse generator can have more or fewer than eight stimulation channels (e.g., 4-, 6-, 16-, 32-, or more stimulation channels). The implantable pulse generator can have one, two, three, four, or more connector ports, for receiving the terminals of the leads.

The ETS 20 may also be physically connected, optionally via the percutaneous lead extensions 28 and external cable 30, to the stimulation leads 12. The ETS 20, which may have similar pulse generation circuitry as the IPG 14, also delivers electrical stimulation energy in the form of, for example, a pulsed electrical waveform to the electrode array 26 in accordance with a set of stimulation parameters. One difference between the ETS 20 and the IPG 14 is that the ETS 20 is often a non-implantable device that is used on a trial basis after the neurostimulation leads 12 have been implanted and prior to implantation of the IPG 14, to test the responsiveness of the stimulation that is to be provided. Any functions described herein with respect to the IPG 14 can likewise be performed with respect to the ETS 20.

The RC 16 may be used to telemetrically communicate with or control the IPG 14 or ETS 20 via a uni- or bi-directional wireless communications link 32. Once the IPG 14 and neurostimulation leads 12 are implanted, the RC 16 may be used to telemetrically communicate with or control the IPG 14 via a uni- or bi-directional communications link 34. Such communication or control allows the IPG 14 to be turned on or off and to be programmed with different stimulation parameter sets. The IPG 14 may also be operated to modify the programmed stimulation parameters to actively control the characteristics of the electrical stimulation energy output by the IPG 14. The CP 18 allows a user, such as a clinician, the ability to program stimulation parameters for the IPG 14 and ETS 20 in the operating room and in follow-up sessions.

The CP 18 may perform this function by indirectly communicating with the IPG 14 or ETS 20, through the RC 16, via a wireless communications link 36. Alternatively, the CP 18 may directly communicate with the IPG 14 or ETS 20 via a wireless communications link (not shown). The stimulation parameters provided by the CP 18 are also used to program the RC 16, so that the stimulation parameters can be subsequently modified by operation of the RC 16 in a stand-alone mode (i.e., without the assistance of the CP 18).

For purposes of brevity, the details of the RC 16, CP 18, ETS 20, and external charger 22 will not be further described herein. Details of exemplary embodiments of these devices are disclosed in U.S. Pat. No. 6,895,280. Other examples of electrical stimulation systems can be found at U.S. Patents Nos. 6,181,969; 6,516,227; 6,609,029; 6,609,032; 6,741,892; 7,949,395; 7,244,150; 7,672,734; and 7,761,165; 7,974,706; 8,175,710; 8,224,450; and 8,364,278; and U.S. Patent Application Publication No. 2007/0150036, as well as the other references cited above.

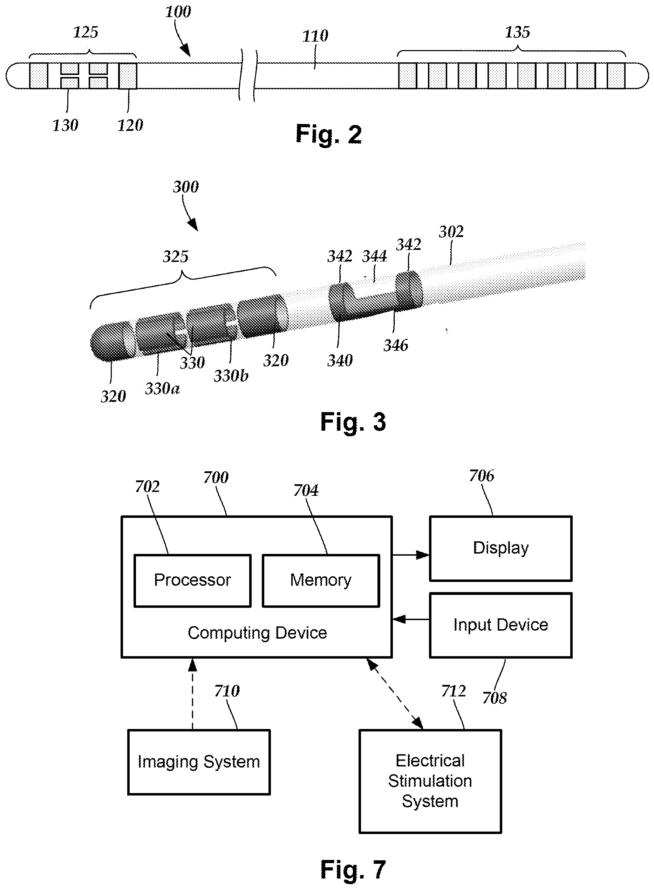

Figure 2 illustrates one embodiment of a lead 110 with electrodes 125 disposed at least partially about a circumference of the lead 110 along a distal end portion of the lead and terminals 135 disposed along a proximal end portion of the lead. The lead 110 can be implanted near or within the desired portion of the body to be stimulated such as, for example, the brain, spinal cord, or other body organs or tissues. In one example of operation for deep brain stimulation, access to the desired position in the brain can be accomplished by drilling a hole in the patient's skull or cranium with a cranial drill (commonly referred to as a burr), and coagulating and incising the dura mater, or brain covering. The lead 110 can be inserted into the cranium and brain tissue with the assistance of a stylet (not shown). The lead 110 can be guided to the target location within the brain using, for example, a stereotactic frame and a microdrive motor system. In some embodiments, the microdrive motor system can be fully or partially automatic. The microdrive motor system may be configured to perform one or more the following actions (alone or in combination): insert the lead 110, advance the lead 110, retract the lead 110, or rotate the lead 110.

In some embodiments, measurement devices coupled to the muscles or other tissues stimulated by the target neurons, or a unit responsive to the patient or clinician, can be coupled to the implantable pulse generator or microdrive motor system. The measurement device, user, or clinician can indicate a response by the target muscles or other tissues to the stimulation or recording electrode(s) to further identify the target neurons and facilitate positioning of the stimulation electrode(s). For example, if the target neurons are directed to a muscle experiencing tremors, a measurement device can be used to observe the muscle and indicate changes in, for example, tremor frequency or amplitude in response to stimulation of neurons. Alternatively, the patient or clinician can observe the muscle and provide feedback.

The lead 110 for deep brain stimulation can include stimulation electrodes, recording electrodes, or both. In at least some embodiments, the lead 110 is rotatable so that the stimulation electrodes can be aligned with the target neurons after the neurons have been located using the recording electrodes.

Stimulation electrodes may be disposed on the circumference of the lead 110 to stimulate the target neurons. Stimulation electrodes may be ring-shaped so that current projects from each electrode equally in every direction from the position of the electrode along a length of the lead 110. In the embodiment of Figure 2, two of the electrodes 125 are ring electrodes 120. Ring electrodes typically do not enable stimulus current to be directed from only a limited angular range around of the lead. Segmented electrodes 130, however, can be used to direct stimulus current to a selected angular range around the lead. When segmented electrodes are used in conjunction with an implantable pulse generator that delivers constant current stimulus, current steering can be achieved to more precisely deliver the stimulus to a position around an axis of the lead (*i.e.,* radial positioning around the axis of the lead). To achieve current steering, segmented electrodes can be utilized in addition to, or as an alternative to, ring electrodes.

The lead 100 includes a lead body 110, terminals 135, and one or more ring electrodes 120 and one or more sets of segmented electrodes 130 (or any other combination of electrodes). The lead body 110 can be formed of a biocompatible, nonconducting material such as, for example, a polymeric material. Suitable polymeric materials include, but are not limited to, silicone, polyurethane, polyurea, polyurethane-urea, polyethylene, or the like. Once implanted in the body, the lead 100 may be in contact with body tissue for extended periods of time. In at least some embodiments, the lead 100 has a cross-sectional diameter of no more than 1.5 mm and may be in the range of 0.5 to 1.5 mm. In at least some embodiments, the lead 100 has a length of at least 10 cm and the length of the lead 100 may be in the range of 10 to 70 cm.

The electrodes 125 can be made using a metal, alloy, conductive oxide, or any other suitable conductive biocompatible material. Examples of suitable materials include, but are not limited to, platinum, platinum iridium alloy, iridium, titanium, tungsten, palladium, palladium rhodium, or the like. Preferably, the electrodes are made of a material that is biocompatible and does not substantially corrode under expected operating conditions in the operating environment for the expected duration of use.

Each of the electrodes can either be used or unused (OFF). When the electrode is used, the electrode can be used as an anode or cathode and carry anodic or cathodic current. In some instances, an electrode might be an anode for a period of time and a cathode for a period of time.

Deep brain stimulation leads may include one or more sets of segmented electrodes. Segmented electrodes may provide for superior current steering than ring electrodes because target structures in deep brain stimulation are not typically symmetric about the axis of the distal electrode array. Instead, a target may be located on one side of a plane running through the axis of the lead. Through the use of a radially segmented electrode array ("RSEA"), current steering can be performed not only along a length of the lead but also around a circumference of the lead. This provides precise three-dimensional targeting and delivery of the current stimulus to neural target tissue, while potentially avoiding stimulation of other tissue. Examples of leads with segmented electrodes include U.S. Patents Nos. 8,473,061; 8,571,665; and 8,792,993; U.S. Patent Application Publications Nos. 2010/0268298; 2011/0005069; 2011/0130803; 2011/0130816; 2011/0130817; 2011/0130818; 2011/0078900; 2011/0238129; 2012/0016378; 2012/0046710; 2012/0071949; 2012/0165911; 2012/197375; 2012/0203316; 2012/0203320; 2012/0203321; 2013/0197424; 2013/0197602; 2014/0039587; 2014/0353001; 2014/0358208; 2014/0358209; 2014/0358210; 2015/0045864; 2015/0066120; 2015/0018915; 2015/0051681; U.S. Patent Applications Serial Nos. 14/557,211 and 14/286,797; and U.S. Provisional Patent Application Serial No. 62/113,291. Segmented electrodes can also be used for other stimulation techniques including, but not limited to, spinal cord stimulation, peripheral nerve stimulation, dorsal root ganglion stimulation, or stimulation of other nerves, muscles, and tissues

In many instances, it is important to identify the rotational orientation of a lead with segmented electrodes when the lead is implanted into the patient (for example, in the brain of a patient.) Knowing the rotational orientation of the lead (and, in particular, the rotational orientation of the individual segmented electrodes) will facilitate determining which segmented electrodes may be situated for stimulating a particular anatomical or physiological tissue target or determining an expected direction of the electrical stimulation field that can be generated by each of the segmented electrodes. It may be difficult to determine orientation radiologically because the segmented electrodes at least longitudinal position will often overlap in a radiological image.

To facilitate radiological identification of rotational orientation, the lead can include a rotationally asymmetric marker made of different material (for example, a conductive material such as metal) from the lead body so that the marker and lead body are radiologically distinguishable. Figure 3 illustrates one example of a distal portion of a lead 300 with a lead body 302 and electrodes 325 including one or more optional ring electrodes 320 and multiple segmented electrodes 330. The lead 300 also includes a marker 340 that is asymmetrically shaped. The marker 340 is made of a material that is substantially different from the material of the lead body 302, particularly, when viewed using a radiological imaging technique, such as CT imaging, so that the marker is radiologically distinguishable from the lead body. In at least some embodiments, the marker 340 is made of metal (such as a pure metal or an alloy) and, in at least some embodiments, is made of the same material as the electrodes 325.

The marker 340 defines one or more optional rings 342 formed around the entire circumference of the lead 300, at least one window 344, and a longitudinal band 346 disposed opposite the window. In at least some embodiments, the longitudinal band 346 of the marker 340 extends around no more than 80%, 75%, 67%, 60%, 50%, 40%, 34%, 30%, 25%, or 20% of the circumference of the lead with the window 344 extending around the remainder of the circumference. To further facilitate the determination of directionality of the marker 340 and ensure that the resulting bulge, described below, will be visible around less than half of the lead, the longitudinal band 346 will extend around less than half the circumference of the lead and may extend around no more than one third or one quarter of the circumference. In at least some embodiments, the longitudinal band 346 of the marker 340 is aligned with at least one of the segmented electrodes 330 (such as segmented electrodes 330a, 330b in the illustrated embodiment of Figure 3.) In the illustrated embodiments, the longitudinal band 346 extends between two rings 342. Examples of other markers that can be used in place of marker 340 can be found in U.S. Provisional Patent Application Serial No. 62/408,392.

As explained in more detail below, radiological imaging, such as computed tomography (CT) imaging, can be used to identify the orientation of the lead due to the asymmetry of the marker. The use of an asymmetrical marker 340 on the lead 300 aids in the identification of the rotational orientation of the lead using imaging techniques, such as CT imaging or other radiological imaging techniques. The marker 340 is asymmetric because it is not rotationally symmetric (in contrast to a ring) with, along at least one circumferential region of the lead, marker material around a portion of the circumference of the lead and absence of marker material (e.g., a marker window) along another portion of the circumference of the lead. Examples of methods and systems for identifying lead orientation using radiological imaging are described in U.S. Provisional Patent Applications Serial Nos. 62/209,001 and 62/212, 775.

In at least some of these methods and systems, actual CT scans for known leads in known orientations is obtained (for example, collected in a database or stored as templates). These CT scans are then used to facilitate determination of orientation of a lead using radiological imaging. However, instead of, or in addition to, using actual CT scans of known leads in known positions or trajectories and in known rotational orientations, simulated CT scans of known leads in known positions or trajectories and in known rotational orientations can be generated and used for lead orientation determinations. For example, a database (or set of templates) of simulated CT scans of known leads in a variety of different orientations can be generated and used. Such simulated CT scans can be generated using any suitable software. In yet other embodiments, a database can include both actual CT scans and simulated CT scans. CT scanner manufacturers often include in their scanning software algorithms for reducing artifacts or otherwise improving images. In at least some embodiments, the simulated CT scans also incorporate in the simulation generation software similar algorithms. In other embodiments, the simulation generation software does not include such algorithms for reducing artifacts or otherwise improving the images. It will be recognized that similar databases or templates can be generated using actual or simulated images for imaging modalities other than CT such as, for example, magnetic resonance imaging (MRI), X-ray, positron emission tomography (PET), or single-photon emission computed tomography (SPECT), or the like. In some embodiments, the position of the actual or simulated image can also be categorized based on which hemisphere the lead is implanted.

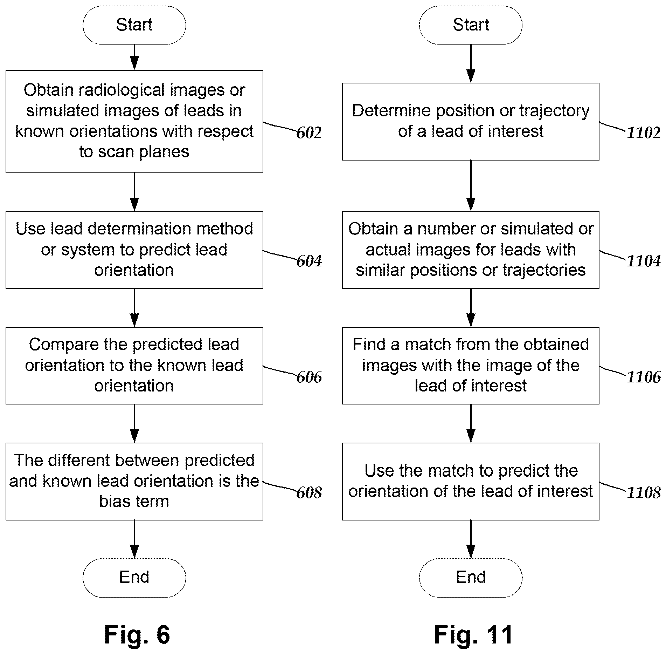

For example, any lead orientation determination method or system, including, but not limited to those discussed below or described in U.S. Provisional Patent Applications Serial Nos. 62/209,001 and 62/212,775
can include the use of simulated CT scans or other simulated images of known leads instead of, or in addition to, actual images of those known leads. Figure 11 illustrates one embodiment of such a method. In step 1102, the position or trajectory of a lead of interest is determined In step 1104, a number of simulated or actual CT scans (or other simulated or actual images) for leads with similar positions or trajectories as the lead of interest is obtained. In step 1106, these CT scans (or other images) from the database can be transformed or otherwise selected to find a match (within a threshold level of matching) to the lead of interest. In step 1108, this transformation or selection can then be used to predict the orientation of the lead of interest.

In at least some embodiments, a system or method includes generating or otherwise obtaining radiological images of an implanted lead 300 with an asymmetric marker 340 that will aid a clinician in making a determination of the rotational orientation of the lead. In at least some embodiments, determining the rotational orientation of the lead can also aid in determining the location of the segmented electrodes relative to anatomical or physiological structures of interest (for example, structures in the brain, spinal cord, or other patient tissues). In at least some embodiments, display of information from one or more radiological (e.g., CT) images in the form of an isosurface image can be used to identify the rotational orientation of the lead 300 using the marker 304. For example, the asymmetric marker 340 of the lead 300 can produce a bulge in an appropriately selected CT isosurface that forms in the direction of the longitudinal band 346 of the marker.

An isosurface image from CT image (or other radiological image) data can be generated, for example, by combining image data from a series of slices and then selecting those portions of the combined image that have the same intensity (or fall within the same narrow band of intensities) or other image parameter. In the example of CT imaging, the intensity is often related to the absorption of x-rays by the material being imaged. For example, a marker 340 made of metal will typically have a higher absorption of x-rays than the lead body 302, which is formed of a polymeric material, resulting in different, and distinguishable, intensities for these components in a CT image. An isosurface image, generated from the corresponding CT images by selecting a single intensity or narrow band of intensities, can be used to identify those portions of the lead that are formed of metal, such as the marker 340.

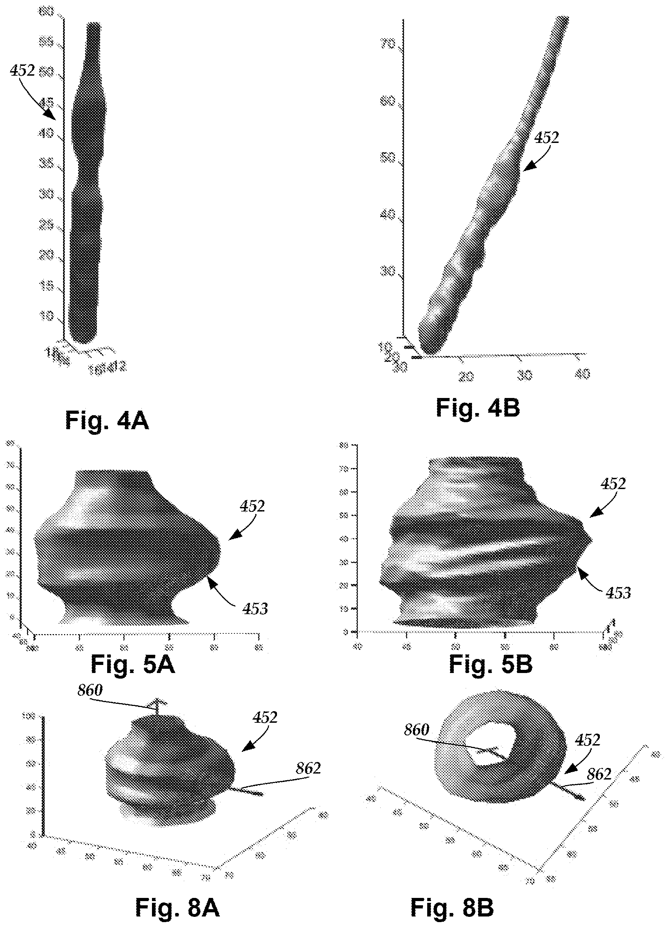

As an example, Figures 4A and 4B illustrate isosurface images from CT scans of a lead with segmented electrodes and an asymmetric marker. In Figure 4A, the lead was aligned along the scanner axis and, in Figure 4B, the lead was aligned at an angle to the scanner axis. The intensities in this CT scan are coded in the standard Hounsfield Units (HU) which in these examples ranges from -1024 to +3072. In this particular example, the isosurface image was drawn at 2000 HU. The slice thickness was 0.6 mm and adjacent slices had 50% overlap.

A distinct bulge 452 of the isosurface in the marker portion is apparent in both isosurface images. Figures 4A and 4B clearly show that the lead marker portion appears to be anisotropic i.e. it has a specific direction. In particular, the metal band of the marker causes a bulge in the isosurface and the opposite side, which corresponds to the absence of metal, does not exhibit this bulge in the isosurface.

Figures 5A and 5B illustrate the isosurface images for the leads of Figures 4A and 4B, respectively, restricted to the marker portion. The isosurface images were generated by resampling the CT data on an isotropic grid aligned with the lead axis, with voxel size 0.1 mm in each direction. Resampling on a grid aligned with the lead axis can facilitate visualization of features that may not be as apparent when the CT slices are aligned at an angle to the lead axis. Resampling on a finer grid may also facilitate visualization. The bulge 452 corresponding to the position of the longitudinal band 346 of the lead marker 340 is clearly observed. In addition, there may be a dent 453 on the marker portion between the bulge of the longitudinal band (right portion of the isosurfaces in Figures 5A and 5B) and the window (left portion of the isosurfaces in Figures 5A and 5B).

In at least some embodiments, the lead orientation determination methods and systems, such as those described in U.S. Provisional Patent Applications Serial Nos. 62/209,001 and 62/212,775, can be enhanced or improved by incorporating a correction that can arise, for example, from the radiological imaging methodology. For example, at least some CT imaging procedures utilize a helical scanning methodology in which the scanner rotates around the patient while the patient is slowly moving longitudinally. Image reconstruction algorithms are employed to account for this helical path, but these algorithms may create a systematic deviation in the orientation determination from the resulting image. In at least some embodiments, the deviation is smallest when the lead is positioned perpendicular to the CT scan planes and is largest when the lead is positioned along the CT scan plane. Methods for accounting for this deviation, however, are not limited to such embodiments. CT scans are used as examples below, but these methods can be applied to other imaging techniques.

Figure 6 illustrates one embodiment of a method for correction of lead orientation determination. In step 602, scans of leads with known orientations of the marker and positioned at different angles to the CT scan planes are obtained. These CT scans can be either actual radiological images or simulated CT scans or any combination thereof. For example, the scans of step 602 can be obtained from a database of actual CT scans of known leads in known positions or trajectories and in known rotational orientations. As another example, the scans of step 602 can be obtained from a database of simulated CT scans of known leads in a variety of different positions or trajectories and different rotational orientations. Such simulated CT scans can be generated using any suitable software. In yet other embodiments, the scans of step 602 can be obtained from a database that includes both actual CT scans and simulated CT scans. CT scanner manufacturers often include in their scanning software algorithms for reducing artifacts or otherwise improving images. In at least some embodiments, the simulated CT scans also incorporate in the simulation generation software similar algorithms. In other embodiments, the simulation generation software does not include such algorithms for reducing artifacts or otherwise improving the images.

Then, in step 604 a lead orientation determination method or system, such as those discussed below or described in U.S. Provisional Patent Applications Serial Nos. 62/209,001 and 62/212,775, is used to predict the rotational orientation of the lead. In at least some embodiments, the lead determination method or system is the same method or system that will be used subsequently to determine the orientation of implanted leads with otherwise unknown orientation. In other embodiments, the correction obtained by this method can be used with more than one different lead determination method or system. For example, the position or trajectory of a lead of interest can be determined, a number of simulated or actual CT scans for leads with similar positions or trajectories as the lead of interest can be obtained from a database, these CT scans from the database can be transformed or otherwise selected to find a match (within a threshold level of matching) to the lead of interest, and this transformation or selection can then be used to predict the rotational orientation of the lead of interest.

In step 606, the predictions from step 604 are compared to the known orientation of the lead. In step 608, the difference between the prediction and the truth is stored as a bias term. In at least some embodiments, this bias term depends on the angle of the lead with the CT planes and a database (e.g., a look-up table) is created with various bias terms and corresponding lead axis-CT plane angles. In at least some embodiments, this bias term depends on the angle of the marker with the CT scanner coordinate axes and a database (e.g., a look-up table) is created with various bias terms and corresponding marker-CT scanner-coordinate-axes angles.

When a new lead is presented with an angle relative to the CT planes that is equal to the angles stored in the database, a lead determination method or system predicts a putative orientation and then uses the bias term to correct this prediction. If the presented lead forms an angle to the CT plane that is not present in the database, the algorithm uses the closest angle from the database or can interpolate the bias terms from entries in the database for other angles to determine a bias term for the presented lead. The interpolation can be weighted with a weighting function that emphasizes angles from the database that are close to the angle exhibited by the presented lead. Any interpolation method can be used including linear and non-linear interpolation methods. A similar database, process, and arrangement can also be used for the angle of the marker relative to the CT scanner coordinate axes, instead of the angle of the lead relative to the CT planes.

In at least some embodiments, the database can also be used to account for other scanning variables such as, for example, slice thickness, pitch, or other factors (or any combination of these factors) that affect the scan. Then, just as the angle of the presented lead is compared with the stored angles, the slice thickness (or other imaging parameter) of the presented scan can be compared with the slice thicknesses (or other imaging parameters) stored in the database. Interpolation can also be used with these factors as well to determine a bias term. When multiple imaging parameters are considered, in some embodiments, a separate bias term is determined for each imaging parameter and combined in any suitable linear or non-linear manner. In other embodiments, the database may be a multi-variable database so that the bias term is selected based on the combination of the imaging parameters. Again, interpolation methods can be used to determine the bias terms.

In at least some embodiments, any of the databases described above can be modified to provide a corrected lead orientation instead of a bias term. As an alternative to the databases described above, the known and predicted lead orientations, along with the angle to the scan plane or to the scanner coordinate axes or other imaging parameters, can be used to produce a relationship (for example, a linear or non-linear equation) that uses the predicted lead orientation and one or more imaging parameters as inputs and produces either the bias term or the corrected lead orientation as an output. Any method of determining the relationship can be used including linear or non-linear extrapolation methods or machine learning methods.

Figure 7 illustrates one embodiment of a system for practicing the invention. The system can include a computing device 700 or any other similar device that includes a processor 702 and a memory 704, a display 706, an input device 708, and, optionally, the electrical stimulation system 712 (such as the system 10 in Figure 1). The system 700 may also optionally include one or more imaging systems 710 (for example, a CT imaging system). In some embodiments, the computing device 700 is part of the imaging system 710. In some embodiments, the computing device 700 is part of the electrical stimulation system 712, such as part of the clinician programmer 18 (Figure 1), remove control 16 (Figure 1), or external trial stimulator 20 (Figure 1). In other embodiments, the computing device 700 is not part of either the electrical stimulation system 712 or imaging system 710.

The computing device 700 can be a computer, tablet, mobile device, or any other suitable device for processing information. The computing device 700 can be local to the user or can include components that are non-local to the computer including one or both of the processor 702 or memory 704 (or portions thereof). For example, in some embodiments, the user may operate a terminal that is connected to a non-local computing device. In other embodiments, the memory can be non-local to the user.

The computing device 700 can utilize any suitable processor 702 including one or more hardware processors that may be local to the user or non-local to the user or other components of the computing device. The processor 702 is configured to execute instructions provided to the processor.

Any suitable memory 704 can be used for the computing device 702. The memory 704 illustrates a type of computer-readable media, namely computer-readable storage media. Computer-readable storage media may include, but is not limited to, nonvolatile, non-transitory, removable, and non-removable media implemented in any method or technology for storage of information, such as computer readable instructions, data structures, program modules, or other data. Examples of computer-readable storage media include RAM, ROM, EEPROM, flash memory, or other memory technology, CD-ROM, digital versatile disks ("DVD") or other optical storage, magnetic cassettes, magnetic tape, magnetic disk storage or other magnetic storage devices, or any other medium which can be used to store the desired information and which can be accessed by a computing device.

Communication methods provide another type of computer readable media; namely communication media. Communication media typically embodies computer-readable instructions, data structures, program modules, or other data in a modulated data signal such as a carrier wave, data signal, or other transport mechanism and include any information delivery media. The terms "modulated data signal," and "carrier-wave signal" includes a signal that has one or more of its characteristics set or changed in such a manner as to encode information, instructions, data, and the like, in the signal. By way of example, communication media includes wired media such as twisted pair, coaxial cable, fiber optics, wave guides, and other wired media and wireless media such as acoustic, RF, infrared, and other wireless media.

The display 706 can be any suitable display device, such as a monitor, screen, display, or the like, and can include a printer. The input device 708 can be, for example, a keyboard, mouse, touch screen, track ball, joystick, voice recognition system, or any combination thereof, or the like.

One or more imaging systems 710 can be used including, but not limited to, MRI, CT, ultrasound, or other imaging systems. The imaging system 710 may communicate through a wired or wireless connection with the computing device 700 or, alternatively or additionally, a user can provide images from the imaging system 710 using a computer-readable medium or by some other mechanism.

The electrical stimulation system 712 can include, for example, any of the components illustrated in Figure 1. The electrical stimulation system 712 may communicate with the computing device 700 through a wired or wireless connection or, alternatively or additionally, a user can provide information between the electrical stimulation system 712 and the computing device 700 using a computer-readable medium or by some other mechanism. In some embodiments, the computing device 700 may include part of the electrical stimulation system, such as, for example, the IPG, CP, RC, ETS, or any combination thereof.

One or more images or imaging data, for example, CT images or imaging data, can be provided to, or generated by, the computing device 700. The images or imaging data can be provided by the imaging system 710 or any other suitable image source.

With respect to lead determination methods that utilize isosurfaces, the computing device can produce an isosurface image, as illustrated in Figures 4A-5B. Alternatively, the isosurface image can be provided to the computing device 700 from the imaging system 710 or any other suitable source. In at least some embodiments, the computing device 700 displays the isosurface image on the display 706. The isosurface image may be based on a user-specified isovalue (or narrow range around the user-specified isovalue) or may be based on a default or predefined isovalue (or narrow range around the isovalue). In some embodiments, the computing device 700 provides a user interface for receiving the user-specified isovalue as an input. In some embodiments, the user interface can include a slider bar, or other mechanism, that allows the user to change the isovalue and see the resulting isosurface image. In at least some embodiments, at low isovalues voxels corresponding to the skull and the brain may be included in the isosurface image. In at least some embodiments, to avoid this, the system may require that the user-specified isovalue meet or exceed a threshold (for example, a threshold of 1000 or 30% of the peak Hounsfield unit or any other suitable value).

In at least some embodiments, the orientation of the marker 340 is more discernible in the isosurface image when the marker is viewed from certain angles. In at least some embodiments, the computing device 700 includes a user interface that has one or more controls to allow the user to rotate the isosurface around one or more axes. Such controls can include, for example, clockwise or counterclockwise (or other) rotational controls, as well as axis selection controls. In some embodiments, the user interface allows the user to rotate the isosurface around one, two, three of the coordinate axes, such as the coordinate axes illustrated in Figures 4A-5B. Figure 8A illustrates the isosurface image of Figure 5A rotated about an axis and Figure 8B illustrates the isosurface image of Figure 5A rotated for top-down view with the longitudinal axis of the lead forming a point in the image. In some embodiments, the user interface allows the user to rotate the isosurface about the longitudinal axis of the lead. In some embodiments, other axes of rotation may also be selected or the user may be allowed to define an axis of rotations. In some embodiments, the user interface has a control (such as a play button) that will continuously rotate the lead around the longitudinal axis of the lead and may optionally include a control to modify the speed of the rotation. In some embodiments, the computing device 700 may allow display of more than one isosurface image (for example, the isosurface image at different angles) so that the user can compare the images. In some embodiments, the computing device 700 may include one or more controls that display the isosurface image at predetermined orientations (for example, a control to produce a top-down view (for example, Figure 8B) of the isosurface image or a control to produce an edge-on view (for example, Figures 4A-5B and 8A)).

In some embodiments, the computing device 700 includes a user interface with lead orientation controls that allow the user to define a direction on the isosurface, for example, a direction that corresponds, in the user's opinion, to the longitudinal band 346 of the marker 340. For example, the computing device can provide the user with two orientation axes 860, 862, as illustrated in Figures 8A and 8B. In at least some embodiments, these orientation axes can be centered on the marker midpoint and with, for example, one orientation axis 860 along the longitudinal axis of the lead. The computing device 700 may select the second orientation axis 862 or may permit the user to select the second orientation axis. In at least some embodiments, the user can rotate or otherwise move the second orientation axis to align the second axis with the bulge 452 corresponding to the longitudinal band 346 of the marker 340. In some embodiments, the computing device 700 may determine an initial orientation of the second orientation axis 862 based on analysis of the original images or imaging data or analysis of the isosurface image (or analysis of any combination of the original images, imaging data, or isosurface image) and place the second orientation axis 862 in that initial orientation on the display device.

In some embodiments, the user interface includes one or more controls that allow the user to utilize the lead isosurface image to manually assign one or more of the location of the lead tip, the location of the lead shaft, the location of one or more electrodes (including segmented electrodes), or any combination thereof.

In some embodiments, the user interface can utilize the images and the identification of the position of the longitudinal band of the marker to then depict the location and orientation of the lead (as, for example, a model of the lead that optionally includes models of the lead electrodes). In at least some embodiments, the user interface may also depict the location and orientation of the lead with respect to anatomical or physiological structures. In some embodiments, the user interface may include controls to provide calculated distances between the different electrodes of the leads and anatomical or physiological structures (for example, brain structures) in the image once the lead marker orientation is determined. Such calculations and depictions may utilize additional images (such as CT or MRI images) to identify or infer locations of the anatomical or physiological structures. The user interface may also include tools provided for measuring distances (for example, a distance between an electrode and a point in the imaged anatomy) using, for example, a ruler or lines. In at least some embodiments, the user interface can also include controls to display the original images (e.g., CT images) from which the isosurface image is derived or additional images obtained or generated from an imaging system. The identification of anatomical and physiological structures is discussed in at least U.S. Patents Nos. 8,326,433; 8,675,945; 8,831,731; 8,849,632; and 8.958,615; U.S. Patent Application Publications Nos. 2009/0287272; 2009/0287273; 2012/0314924; 2013/0116744; 2014/0122379; and 2015/0066111; and U.S. Provisional Patent Application Serial No. 62/030,655.

In at least some embodiments, the user interface may include controls to allow the user to zoom onto any selected portion of the lead, including the marker, within the isosurface image or zoom away from the lead to obtain a view of a larger portion of the lead in the isosurface image. This may facilitate observation of the marker shape.

In addition, the user interface may include one or more of the following: a control for resampling the image data on a finer grid; a control to align the lead axis with one of the coordinate axes (or this may be performed automatically as a default); a control to change lighting or color or both to make the isosurface shape more discernible, or any combination of these and the other controls described herein.

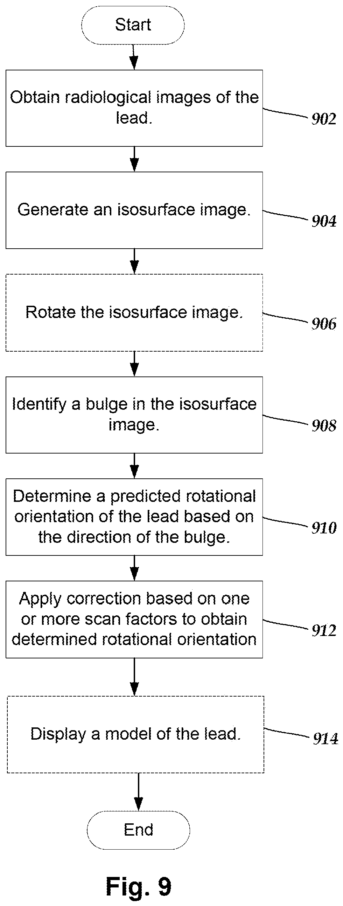

Figure 9 illustrates one method of determining the rotational orientation of a lead, such as lead 300. In step 902, radiological images of the lead are obtained. The lead has an asymmetric marker as described above. In step 904, an isosurface image is generated from the radiological images and displayed on a display device. In optional step 906, the isosurface image can be rotated on the display. In step 908, a bulge in the isosurface image corresponding to the longitudinal band of the marker is identified. In step 910, a predicted rotational orientation of the lead is determined based on the direction of the bulge in the isosurface image. In step 912, a correction is applied to the predicted rotational orientation based on one or more imaging parameters to obtain the determined rotational orientation. The correction can be determined as described above with respect to Figure 6 and can be, for example, a bias term that is applied to the predicted rotational orientation. The one or more imaging parameters can be, for example, the angle of the lead with respect to the scan plane, the angle of the maker with respect to the scanner coordinate axes, slice thickness, pitch, or the like or any combination thereof. In optional step 914, a model of the lead is displayed based on the determined rotational orientation. The steps of this method may be modified to incorporate any of the other features of the computing device or user interface described above. Steps of this method can be performed by the computing device and, in some instances, in response to user input or command.

Another method of determining lead orientation using radiological imaging utilizes templates. Examples of systems for performing such methods can be found in, for example, U.S. Provisional Patent Application Serial No. 62/212,775.

The templates are templates of reference leads. The reference leads may be identical to, or of the same type of, the lead for which the orientation is to be determined. The template may be constructed using image data of the reference lead when the reference lead is substantially aligned with an imaging axis, and positioned with a specified and known rotational orientation.

In some embodiments, the image data used to construct the template is actual image data in one or more known orientations and may be obtained from the same type of imaging system used to produce the image of the lead for which the orientation is to be determined. In other embodiments, the image data may be simulated image data that simulates a scan of the lead in one or more known orientations. In yet other embodiments, any combination of actual and simulated image data can be used.

The template may include a reference data cube (X_{R}) of the marker and a reference marker direction vector (v_{R}) indicative of the specified rotational orientation of the lead about the longitudinal axis of the lead. The reference data cube (X_{R}) may be a selected portion of the image data of the marker (such as marker 340 of Figure 3) of a reference lead. In an example, the reference data cube (X_{R}) may be a three-dimensional (3D) data array of a volume of the marker image. In some examples, the template may include other forms of data representation, in lieu of the data cube (X_{R}), that represent anisotropy of the marker image, such as an isosurface. The reference marker direction vector (v_{R}) may be generated using the image data of the marker image.

As an example, a marker recognition circuit may identify the marker from the image data of the lead. The marker recognition circuit may further use the image data of the identified marker to produce a target data cube (X_{T}) of the marker. Similar to the reference data cube X_{R} extracted from the marker image of the reference lead with a specified and known lead orientation, the target data cube X_{T} may be extracted from a selected portion such as the marker image of the target lead with a target, unknown lead orientation. In an example, X_{T} may have a similar data structure as X_{R}, such as a 3D data array of a volume of the marker image corresponding to the target lead.

As an example, a data registration circuit may register the reference data cube X_{R} to the target data cube X_{T}. Because both X_{R} and X_{T} have the same image data format and constructed into a similar data structure, the data registration circuit may produce a transformation operator Φ for transforming X_{R} into a transformed reference data cube Φ(X_{R}), or a "registered reference data cube." The transformation operator Φ may be an affine transformation. The affine transformation may include rigid transformations that preserve the distance, such as one or any combination of a translation, a rotation, or a reflection operation; or non-rigid transformations such as one or any combination of stretching, shrinking, or model-based transformations such as radial basis functions, splines, or finite element model. In some embodiments, the transformation may include both the rigid transformation to bring reference data cube X_{R} in global alignment with the size and orientation of the target data cube X_{T}, and the non-rigid transformation to reduce the local geometric discrepancies by aligning the reference data cube X_{R} with the target data cube X_{T}.

By registration, the transformed reference data cube Φ(X_{R}) may be in a coordinate system similar to that of the target data cube X_{T}. The data registration circuit may determine the transformation operator Φ as one that causes the transformed reference data cube Φ(X_{R}) to match the target data cube X_{T} within a specified margin. In an example, the transformation operator Φ may minimize the multidimensional distance between Φ(X_{R}) and X_{T}, such as when the distance falls below a specified threshold. Examples of the distance measure may include L1 norm, L2 norm (i.e., Euclidian distance), infinite norm, other norm in the normed vector space, or a dissimilarity measure between Φ(X_{R}) and X_{T} such as correlation coefficient, mutual information, or ratio image uniformity.

As an example, an estimator circuit may estimate the orientation, including a rotational orientation, of the lead using the reference marker direction vector *v*_{R} of the template and the transformation operator Φ as produced by the marker recognition circuit. In an example, the estimator circuit may apply the transformation operator Φ to the reference direction vector *v*_{R} to produce an estimated marker direction vector *ṽ_{T}* = Φ(*v*_{R}). The estimated marker direction vector *ṽ_{T}* may be indicative of the rotational orientation of the lead relative to an imaging axis used for producing the image data of the at least a portion of the lead.

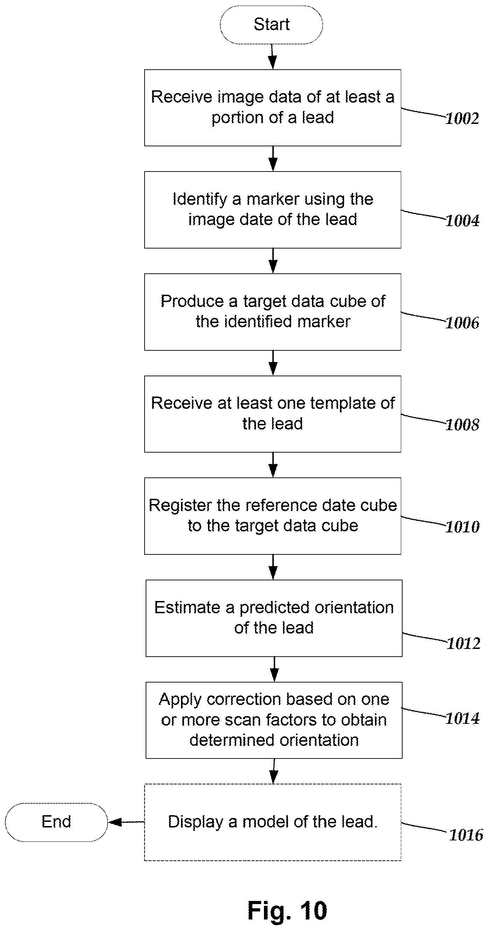

Figure 10 illustrates one embodiment of a method for determining an orientation of a lead. In step 1002, image data of at least a portion of a lead is received, such as from an imaging system such as an X-ray machine, a CT scanner, a MRI scanner, a positron emission tomography (PET) scanner, or a single-photon emission computed tomography (SPECT) scanner, among others. Alternatively, the image data may be received from a machine-readable medium. The lead is positioned in target tissue structures with a target, unknown lead orientation. In an example, the image data may include CT scan image of at least a portion of the lead including image data of the marker.

In step 1004, the marker may be identified using the image data of the lead. The input image of the lead may be segmented, and a characteristic anisotropic shape of the marker portion on the lead may be identified. In step 1006, a target data cube X_{T}, corresponding to the identified marker on the lead, may be produced. Lead tip and lead shaft of the lead may be automatically, or at least based on a user's input, identified using the image segments of the lead. A lead axis may be detected such as by joining the identified lead tip and lead shaft. The target data cube X_{T} may then be produced using image data of the marker and the detected lead axis. The target data cube X_{T} may be sized, shaped, and oriented to contain the marker image and be axially aligned with the detected lead axis. Alternatively, the target data cube X_{T} may be sized, shaped, and oriented to cover only the bulge of the marker corresponding to the marker band, or the entire lead. Depending on the property of the image of the lead, in some example, the target data cube X_{T} may be a 2D data array. In an example, the image data within the target data cube may be resampled to have a higher spatial resolution than that of the image of the lead.

In step 1008, at least one template of the lead may be received, such as from a machine-readable medium or a template formation circuit. The reference lead may be identical to, or of the same type of, the lead used for producing the image data. The template may be constructed using image data of the lead when the lead is substantially aligned with an imaging axis and positioned with a specified and known rotational orientation. The image data may be actual image data or simulated image data or any combination thereof. The template may include a reference data cube (X_{R}) of the marker and a reference marker direction vector (*v*_{R}) indicative of the specified rotational orientation of the lead about the longitudinal axis of the lead. The reference data cube X_{R}, similar to the target data cube X_{T}, may be a selected portion of the image data extracted from the marker image, and has similar data structure as the target data cube X_{T}, such as a 3D data array. In some examples, in lieu of the reference data cube X_{R} and the target data cube X_{T}, other forms of data representation that represent anisotropy of the marker image can be used. For example, an isosurface of the identified marker band can be produced and an isosurface of the marker of the template can be received.

In step 1010, the reference data cube X_{R} may be registered to the target data cube X_{T}, to produce a transformation operator (Φ) for transforming X_{R} into a transformed reference data cube Φ (X_{R}), or a "registered reference data cube." The transformation may include an affine transformation such as a rigid transformation (such as one or any combination of translation, a rotation, or a reflection operation), non-rigid transformation (such as one or any combination of stretching, shrinking, or model-based transformations), or a combination of rigid and non-rigid transformations. The transformation operator Φ may be determined when the registered reference data cube Φ (X_{R}) matches the target data cube X_{T} within a specified margin, such as when multidimensional distance or a dissimilarity measure between Φ(X_{R}) and X_{T} falling below a specified threshold.

In step 1012, a predicted orientation of the direction lead may be estimated using the reference marker direction vector *v*_{R} and the transformation operator Φ. In an example, the transformation operator Φ may be applied to the reference direction vector *v*_{R} to produce an estimated marker direction vector *ṽ_{T}* = Φ(*v*_{R}), which is indicative of the rotational orientation of the lead relative to an imaging axis used for producing the image data of the at least a portion of the lead.

In step 1014, a correction is applied to the predicted orientation based on one or more imaging parameters to obtain the determined orientation. The correction can be determined as described above with respect to Figure 6 and can be, for example, a bias term that is applied to the predicted orientation. The one or more imaging parameters can be, for example, the angle of the lead with respect to the scan plane, the angle of the maker with respect to the scanner coordinate axes, slice thickness, pitch, or the like or any combination thereof.

In step 1016, a graphical representation or model of the estimated orientation of the lead, represented by *ṽ_{T},* may be produced, and displayed. Other information, including the target marker, the reference marker of the template, or the reference marker band direction vector, may also be displayed.

In any of the methods of Figures 9 and 10, the determined orientation of the lead may be used to produce a recommendation of lead positioning, and providing directional electrostimulation to the body tissue using the two or more directional electrodes on the lead oriented at least according to the determined rotational orientation.

In some examples, the method 1000 may be modified to perform orientation estimation based on multi-atlas image registration. For example, in step 1008 two or more templates of the lead, {Template 1, Template 2, ..., Template K}, may be received, each template including a respective reference data cube X_{R} and the corresponding reference direction vector *v*_{R}. In step 1010, the reference data cubes {X_{R1}, X_{R2}, ..., X_{RK}} associated with the respective two or more templates may be registered to the target data cube X_{T}, and the corresponding transformation operators {Φ₁, Φ₂, ..., Φ_{K}} can be produced. In step 1012, two or more estimated marker direction vectors {*ṽ*_{*T*1}, *ṽ*_{*T*2}, *...*, *ṽ_{TK}*} of the lead may be estimated, such that *ṽ_{Ti}* = Φᵢ(*v*_{Ri}) for at least some of the templates. A combined estimate *ṽ_{T}* of the marker direction vector rotational orientation of the direction lead using a fusion function *f* of at least some of the two or more estimated direction vectors {*ṽ*_{*T*1}, *ṽ*_{*T*2}, ..., *ṽ_{TK}*}. A confidence bound of the estimated rotational orientation of the lead may also be estimated using the two or more estimated marker direction vectors. This rotational orientation can then be corrected, in step 1014, to obtain the determined orientation.

The methods and systems described herein may be embodied in many different forms and should not be construed as limited to the embodiments set forth herein. Accordingly, the methods and systems described herein may take the form of an entirely hardware embodiment, an entirely software embodiment or an embodiment combining software and hardware aspects. Systems referenced herein typically include memory and typically include methods for communication with other devices including mobile devices. Methods of communication can include both wired and wireless (e.g., RF, optical, or infrared) communications methods and such methods provide another type of computer readable media; namely communication media. Wired communication can include communication over a twisted pair, coaxial cable, fiber optics, wave guides, or the like, or any combination thereof. Wireless communication can include RF, infrared, acoustic, near field communication, Bluetooth™, or the like, or any combination thereof.

It will be understood that each of the methods disclosed herein, can be implemented by computer program instructions. These program instructions may be provided to a processor to produce a machine, such that the instructions, which execute on the processor, create means for implementing the actions specified in the flowchart block or blocks disclosed herein. The computer program instructions may be executed by a processor to cause a series of operational steps to be performed by the processor to produce a computer implemented process. The computer program instructions may also cause at least some of the operational steps to be performed in parallel. Moreover, some of the steps may also be performed across more than one processor, such as might arise in a multi-processor computer system. In addition, one or more processes may also be performed concurrently with other processes, or even in a different sequence than illustrated without departing from the scope of the invention.

The computer program instructions can be stored on any suitable computer-readable medium including, but not limited to, RAM, ROM, EEPROM, flash memory or other memory technology, CD-ROM, digital versatile disks ("DVD") or other optical storage, magnetic cassettes, magnetic tape, magnetic disk storage or other magnetic storage devices, or any other medium which can be used to store the desired information and which can be accessed by a computing device.

## Claims

1. A method for identifying a rotational orientation of an implanted electrical stimulation lead (12, 100, 300), the method comprising:
obtaining a plurality of radiological images of the lead (12, 100, 300) generated using an imaging technique, the lead comprising a lead body (110, 302), a plurality of segmented electrodes (130, 330) disposed along a distal portion of the lead body, and an asymmetric marker (340) disposed along the distal portion of the lead body and comprising a longitudinal band (346) that extends around a portion of a circumference of the lead body and defines a marker window (344) extending around a remainder of the circumference of the lead body and opposite the longitudinal band, wherein the asymmetric marker and lead body are distinguishable from each other in the radiological images, wherein each segmented electrode extends around no more than 50% of a circumference of the lead body;
generating (904) an isosurface image from the plurality of radiological images and displaying the isosurface image on a display device, wherein the isosurface image comprises an image of the longitudinal band of the marker;
identifying (908) a bulge (452) in the isosurface image corresponding the longitudinal band of the marker;
determining (910) a predicted rotational orientation of the lead based on the bulge in the isosurface image; and
determining (912) a rotational orientation of the lead by correcting the predicted rotational orientation of the lead based on at least one parameter of the imaging technique used to generate the radiological images.

2. The method of claim 1, further comprising displaying (914), on the display device, a model of at least the distal portion of the lead oriented along the rotational orientation.

3. The method of any one of claims 1 or 2, further comprising rotating (906) the isosurface image on the display device in response to a user command.

4. A method for identifying a rotational orientation of an implanted electrical stimulation lead (12, 100, 300), the method comprising:
receiving (1002) image data of at least a portion of the lead including image data of a marker (340), wherein the image data is generated using an imaging technique;
receiving (1008) at least one template of the lead having a specified rotational orientation, the at least one template including (1) a reference data cube (X_{R}) of the marker and (2) a reference marker direction vector (*v*_{R}) indicative of the specified rotational orientation of the lead about the longitudinal axis;
producing a target data cube (X_{T}) of the marker using the image data of the marker;
registering (1010) the reference data cube (X_{R}) to the target data cube (X_{T}) to produce a transformation operator (Φ) for transforming X_{R} into a transformed reference data cube Φ(X_{R}) that matches X_{T} within a specified margin;
estimating (1012) a predicted rotational orientation of the lead using the reference marker direction vector (*v*_{R}) and the determined transformation operator (Φ); and
determining (1014) a rotational orientation of the lead by correcting the predicted rotational orientation of the lead based on at least one parameter of the imaging technique used to generate the image data.

5. The method of claim 4, wherein the at least one template of the lead having a specified rotational orientation is generated from an actual image of a lead or from a simulated image of a lead.

6. The method of any one of claims 4 or 5, wherein estimating the predicted rotational orientation of the lead includes applying the determined transformation operator (Φ) to the reference direction vector (*v*_{R}) to produce an estimated marker direction vector (*ṽ_{T}*) indicative of the predicted rotational orientation of the lead relative to an imaging axis used for producing the image data of the at least a portion of the lead.

7. The method of any one of claims 4-6, wherein:
registering (1010) the reference data cube (X_{R}) to the target data cube (X_{T}) includes performing a multi-atlas registration of respective reference data cubes associated with the two or more templates to the target data cube, to produce respective transformation operators corresponding to the two or more templates; and
estimating the predicted rotational orientation of the lead includes estimating the predicted rotational orientation using a combination of the two or more estimated marker direction vectors estimated using reference direction vectors and respective transformation operators.

8. The method of any one of claims 1-7, wherein the at least one parameter comprises either a) an angle of the lead with respect to scan planes of the imaging technique or b) an angle of the asymmetric marker with respect to scanner coordinate axes of the imaging technique.

9. The method of claim 8, wherein correcting the predicted rotational orientation comprises applying a bias term to the predicted rotational orientation based on either a) the angle of the lead with respect to the scan planes of the imaging technique or b) the angle of the asymmetric marker with respect to the scanner coordinate axes of the imaging technique.

10. The method of claim 9, wherein applying the bias term comprises obtaining the bias term from a database.

11. The method of claim 10, wherein obtaining the bias term from a database comprises determining the bias term by interpolating entries of the database.

12. The method of claim 8, wherein correcting the predicted rotational orientation comprises correcting the predicted rotational orientation using a relationship between the predicted rotational orientation and the angle of the lead with respect to the scan planes of the imaging technique determined by imaging leads at known orientations using the imaging technique.

13. The method of any one of claims 1-12, wherein the at least one parameter comprises a slice thickness of the imaging technique or a pitch of the scan planes of the imaging technique.

14. A system (10) for identifying a rotational orientation of an implanted electrical stimulation lead (12, 100, 300), the system comprising:
a computer processor (18) configured and arranged to perform the method of any one of claims 1-13.

15. A non-transitory computer-readable medium having computer executable instructions stored thereon that, when executed by a processor, cause the processor to perform the method of any one of claims 1-13.

## Patentansprüche

1. Verfahren zum Erkennen einer Drehausrichtung einer implantierten elektrischen Stimulationsleitung (12, 100, 300), wobei das Verfahren aufweist:
Beziehen von mehreren, mit einem Bildgebungsverfahren erzeugten radiologischen Bildern der Leitung (12, 100, 300), wobei die Leitung einen Leitungskörper (110, 302), mehrere segmentierte Elektroden (130, 330), die über einen distalen Abschnitt des Leitungskörpers hinweg angeordnet sind, und einen asymmetrischen Marker (340) aufweist, der entlang des distalen Abschnitts des Leitungskörpers hinweg angeordnet ist und einen Längsstreifen (346) aufweist, der sich um einen Teil eines Umfangs des Leitungskörpers erstreckt und ein Markerfenster (344) definiert, das sich um den verbleibenden Umfang des Leitungskörpers und gegenüber dem Längsstreifen erstreckt, wobei der asymmetrische Marker und der Leitungskörper in den radiologischen Bildern voneinander unterscheidbar sind, wobei sich jede segmentierte Elektrode über höchstens 50 % eines Umfangs des Leitungskörpers erstreckt;
Erzeugen (904) eines Isoflächenbildes aus den mehreren radiologischen Bildern und Anzeigen des Isoflächenbildes auf einem Anzeigegerät, wobei das Isoflächenbild ein Bild des Längsstreifens des Markers umfasst;
Erkennen (908) einer Ausbauchung (452) in dem Isoflächenbild, das dem Längsstreifen des Markers entspricht;
Bestimmen (910) einer vorhergesagten Drehausrichtung der Leitung anhand der Ausbauchung im Isoflächenbild; und
Bestimmen (912) einer Drehausrichtung der Leitung durch Korrigieren der vorhergesagten Drehausrichtung der Leitung anhand von mindestens einem Parameter des zum Erzeugen der radiologischen Bilder verwendeten Bildgebungsverfahrens.

2. Verfahren nach Anspruch 1, ferner aufweisend das Anzeigen (914) eines in der Drehausrichtung ausgerichteten Modells von mindestens dem distalen Abschnitt der Leitung auf dem Anzeigegerät.

3. Verfahren nach einem der Ansprüche 1 oder 2, ferner aufweisend das Drehen (906) des Isoflächenbildes auf dem Anzeigegerät als Reaktion auf einen Benutzerbefehl.

4. Verfahren zum Erkennen einer Drehausrichtung einer implantierten elektrischen Stimulationsleitung (12, 100, 300), wobei das Verfahren aufweist:
Empfangen (1002) von Bilddaten von mindestens einem Abschnitt der Leitung, der Bilddaten eines Markers (340) umfasst, wobei die Bilddaten unter Verwendung eines Bildgebungsverfahrens erzeugt werden;
Empfangen (1008) mindestens einer Vorlage der Leitung mit einer vorgegebenen Drehausrichtung, wobei die mindestens eine Vorlage (1) einen Referenzdatenwürfel (X_{R}) des Markers und (2) einen Referenzmarkerrichtungsvektor (*v*_{R}) umfasst, der die vorgegebene Drehausrichtung der Leitung um die Längsachse angibt;
Herstellen eines Zieldatenwürfels (X_{T}) des Markers mit den Bilddaten des Markers;
Registrieren (1010) des Referenzdatenwürfels (X_{R}) mit dem Zieldatenwürfel (X_{T}), um einen Transformationsoperator (Φ) zum Umwandeln von X_{R} in einen transformierten Referenzdatenwürfel Φ(X_{R}) zu erzeugen, der innerhalb einer vorgegebenen Spanne X_{T} entspricht.
Schätzen (1012) einer vorhergesagten Drehausrichtung der Leitung mit dem Referenzmarkerrichtungsvektor (*v*_{R}) und dem bestimmten Transformationsoperator (Φ); und
Bestimmen (1014) einer Drehausrichtung der Leitung durch Korrigieren der vorhergesagten Drehausrichtung der Leitung anhand von mindestens einem Parameter des zum Erzeugen der Bilddaten verwendeten Bildgebungsverfahrens.

5. Verfahren nach Anspruch 4, wobei die mindestens eine Vorlage der Leitung mit einer vorgegebenen Drehausrichtung aus einem tatsächlichen Bild einer Leitung oder aus einem simulierten Bild einer Leitung erzeugt wird.

6. Verfahren nach einem der Ansprüche 4 oder 5, wobei das Schätzen der vorhergesagten Drehausrichtung der Leitung das Anwenden des bestimmten Transformationsoperators (Φ) auf den Referenzrichtungsvektor (*v*_{R}) umfasst, um einen geschätzten Markerrichtungsvektor (ṽ_{T}) zu erzeugen, der die vorhergesagte Drehausrichtung der Leitung in Bezug auf eine Bildgebungsachse angibt, die zum Erzeugen der Bilddaten für mindestens einen Abschnitt der Leitung verwendet wird.

7. Verfahren nach einem der Ansprüche 4-6, wobei:
das Registrieren (1010) des Referenzdatenwürfels (X_{R}) mit dem Zieldatenwürfel (X_{T}) das Durchführen einer Multi-Atlas-Registrierung von jeweiligen Referenzdatenwürfeln umfasst, die mit den zwei oder mehreren Vorlagen verbunden sind, mit dem Zieldatenwürfel, um jeweils Transformationsoperatoren zu erzeugen, die den zwei oder mehreren Vorlagen entsprechen; und
das Schätzen der vorhergesagten Drehausrichtung der Leitung das Schätzen der vorhergesagten Drehausrichtung mit einer Kombination von zwei oder mehr geschätzten Markerrichtungsvektoren umfasst, die mit Referenzrichtungsvektoren und jeweiligen Transformationsoperatoren geschätzt wurden.

8. Verfahren nach einem der Ansprüche 1-7, wobei der mindestens eine Parameter entweder a) einen Winkel der Leitung zu Scan-Ebenen des Bildgebungsverfahrens oder b) einen Winkel des asymmetrischen Markers zu Scanner-Koordinatenachsen des Bildgebungsverfahrens aufweist.

9. Verfahren nach Anspruch 8, wobei das Korrigieren der vorhergesagten Drehausrichtung das Anwenden eines Richtungsterms auf die vorhergesagte Drehausrichtung auf Basis von entweder a) dem Winkel der Leitung zu den Scan-Ebenen des Bildgebungsverfahrens oder b) dem Winkel des asymmetrischen Markers zu Scanner-Koordinatenachsen des Bildgebungsverfahrens aufweist.

10. Verfahren nach Anspruch 9, wobei das Anwenden des Richtungsterms das Beziehen des Richtungsterms aus einer Datenbank aufweist.

11. Verfahren nach Anspruch 10, wobei das Beziehen des Richtungsterms aus einer Datenbank das Bestimmen des Richtungsterms durch Interpolieren von Einträgen der Datenbank aufweist.

12. Verfahren nach Anspruch 8, wobei das Korrigieren der vorhergesagten Drehausrichtung das Korrigieren der vorhergesagten Drehausrichtung mit einer Beziehung zwischen der vorhergesagten Drehausrichtung und dem Winkel der Leitung zu den Scan-Ebenen des Bildgebungsverfahrens aufweist, der durch Abbilden der Leitungen mit bekannten Ausrichtungen mittels des Bildgebungsverfahrens bestimmt wurde.

13. Verfahren nach einem der Ansprüche 1-12, wobei der mindestens eine Parameter eine Schichtdicke des Bildgebungsverfahrens oder einen Abstand der Scan-Ebenen des Bildgebungsverfahrens aufweist.

14. System (10) zum Erkennen einer Drehausrichtung einer implantierten elektrischen Stimulationsleitung (12, 100, 300), wobei das System aufweist:
einen Computerprozessor (18), der zum Durchführen des Verfahrens nach einem der Ansprüche 1-13 ausgebildet und eingerichtet ist.

15. Nicht-transitorisches computerlesbares Medium, auf dem computerausführbare Anweisungen gespeichert sind, die bei Ausführung durch einen Prozessor den Prozessor zum Durchführen des Verfahrens nach einem der Ansprüche 1-13 veranlassen.

## Revendications

1. Procédé destiné à identifier une orientation de rotation d'une sonde de stimulation électrique implantée (12, 100, 300), le procédé comprenant les étapes ci-dessous consistant à :
obtenir une pluralité d'images radiologiques de la sonde (12, 100, 300) générées en utilisant une technique d'imagerie, la sonde comprenant un corps de sonde (110, 302), une pluralité d'électrodes segmentées (130, 330) disposées le long d'une partie distale du corps de sonde, et un marqueur asymétrique (340) disposé le long de la partie distale du corps de sonde et comprenant une bande longitudinale (346) qui s'étend autour d'une partie d'une circonférence du corps de sonde et définit une fenêtre de marqueur (344) s'étendant autour d'un reste de la circonférence du corps de sonde et à l'opposé de la bande longitudinale, dans lequel le marqueur asymétrique et le corps de sonde peuvent être distingués l'un de l'autre dans les images radiologiques, dans lequel chaque électrode segmentée s'étend autour de 50 % tout au plus d'une circonférence du corps de sonde ;
générer (904) une image d'isosurface à partir de la pluralité d'images radiologiques et afficher l'image d'isosurface sur un dispositif d'affichage, dans lequel l'image d'isosurface comprend une image de la bande longitudinale du marqueur ;
identifier (908) un renflement (452) dans l'image d'isosurface correspondant à la bande longitudinale du marqueur ;
déterminer (910) une orientation de rotation prédite de la sonde sur la base du renflement dans l'image d'isosurface ; et
déterminer (912) une orientation de rotation de la sonde en corrigeant l'orientation de rotation prédite de la sonde sur la base d'au moins un paramètre de la technique d'imagerie utilisée pour générer les images radiologiques.

2. Procédé selon la revendication 1, comprenant en outre l'étape consistant à afficher (914), sur le dispositif d'affichage, un modèle d'au moins la partie distale de la sonde orientée selon l'orientation de rotation.

3. Procédé selon l'une quelconque des revendications 1 et 2, comprenant en outre l'étape consistant à tourner (906) l'image d'isosurface sur le dispositif d'affichage en réponse à une instruction d'utilisateur.

4. Procédé d'identification d'une orientation de rotation d'une sonde de stimulation électrique implantée (12, 100, 300), le procédé comprenant les étapes ci-dessous consistant à :
recevoir (1002) des données d'image d'au moins une partie de la sonde incluant des données d'image d'un marqueur (340), dans lequel les données d'image sont générées en utilisant une technique d'imagerie ;
recevoir (1008) au moins un modèle de la sonde présentant une orientation de rotation spécifiée, ledit au moins un modèle incluant (1) un cube de données de référence (X_{R}) du marqueur et (2) un vecteur de direction de marqueur de référence (*v*_{R}) indicatif de l'orientation de rotation spécifiée de la sonde autour de l'axe longitudinal ;
produire un cube de données cible (X_{T}) du marqueur en utilisant les données d'image du marqueur ;
enregistrer (1010) le cube de données de référence (X_{R}) sur le cube de données cible (X_{T}) en vue de produire un opérateur de transformation (Φ) pour transformer le cube X_{R} en un cube de données de référence transformé Φ(X_{R}) qui correspond au cube X_{T} dans une marge spécifiée ;
estimer (1012) une orientation de rotation prédite de la sonde en utilisant le vecteur de direction de marqueur de référence (*v*_{R}) et l'opérateur de transformation déterminé (Φ) ; et
déterminer (1014) une orientation de rotation de la sonde en corrigeant l'orientation de rotation prédite de la sonde sur la base d'au moins un paramètre de la technique d'imagerie utilisée pour générer les données d'image.

5. Procédé selon la revendication 4, dans lequel ledit au moins un modèle de la sonde présentant une orientation de rotation spécifiée est généré à partir d'une image réelle d'une sonde ou d'une image simulée d'une sonde.

6. Procédé selon l'une quelconque des revendications 4 et 5, dans lequel l'étape d'estimation de l'orientation de rotation prédite de la sonde inclut l'étape consistant à appliquer l'opérateur de transformation déterminé (Φ) au vecteur de direction de référence (*v*_{R}) en vue de produire un vecteur de direction de marqueur estimé (*ṽ_{T}*) indicatif de l'orientation de rotation prédite de la sonde par rapport à un axe d'imagerie utilisé en vue de produire les données d'image de ladite au moins une partie de la sonde.

7. Procédé selon l'une quelconque des revendications 4 à 6, dans lequel :
l'étape d'enregistrement (1010) du cube de données de référence (X_{R}) sur le cube de données cible (X_{T}) inclut l'étape consistant à mettre en œuvre un enregistrement multi-atlas des cubes de données de référence respectifs associés aux deux modèles ou plus sur le cube de données cible, en vue de produire des opérateurs de transformation respectifs correspondant aux deux modèles ou plus ; et
l'étape d'estimation de l'orientation de rotation prédite de la sonde consiste à estimer l'orientation de rotation prédite en utilisant une combinaison de deux vecteurs de direction de marqueur estimés ou plus qui ont été estimés en utilisant des vecteurs de direction de référence et des opérateurs de transformation respectifs.

8. Procédé selon l'une quelconque des revendications 1 à 7, dans lequel ledit au moins un paramètre comprend soit a) un angle de la sonde par rapport à des plans de balayage de la technique d'imagerie, soit b) un angle du marqueur asymétrique par rapport à des axes de coordonnées de scanneur de la technique d'imagerie.

9. Procédé selon la revendication 8, dans lequel l'étape de correction de l'orientation de rotation prédite comprend l'étape consistant à appliquer un terme de biais à l'orientation de rotation prédite sur la base soit a) de l'angle de la sonde par rapport aux plans de balayage de la technique d'imagerie, soit b) de l'angle du marqueur asymétrique par rapport aux axes de coordonnées de scanneur de la technique d'imagerie.

10. Procédé selon la revendication 9, dans lequel l'étape d'application du terme de biais comprend l'étape consistant à obtenir le terme de biais à partir d'une base de données.

11. Procédé selon la revendication 10, dans lequel l'étape d'obtention du terme de biais à partir d'une base de données comprend l'étape consistant à déterminer le terme de biais en interpolant des entrées de la base de données.

12. Procédé selon la revendication 8, dans lequel l'étape de correction de l'orientation de rotation prédite consiste à corriger l'orientation de rotation prédite en utilisant une relation entre l'orientation de rotation prédite et l'angle de la sonde par rapport aux plans de balayage de la technique d'imagerie déterminé en imageant des sondes à des orientations connues en utilisant la technique d'imagerie.

13. Procédé selon l'une quelconque des revendications 1 à 12, dans lequel ledit au moins un paramètre comprend une épaisseur de tranche de la technique d'imagerie ou un pas des plans de balayage de la technique d'imagerie.

14. Système (10) destiné à identifier une orientation de rotation d'une sonde de stimulation électrique implantée (12, 100, 300), le système comprenant :
un processeur informatique (18) configuré et agencé de manière à mettre en œuvre le procédé selon l'une quelconque des revendications 1 à 13.

15. Support non transitoire lisible par ordinateur sur lequel sont stockées des instructions exécutables par ordinateur qui, lorsqu'elles sont exécutées par un processeur, amènent le processeur à mettre en œuvre le procédé selon l'une quelconque des revendications 1 à 13.
